# EUROPEAN PATENT APPLICATION

(11) **EP 4 671 767 A2**
(43) Date of publication of application: **31.12.2025**
(21) Application number: 25198900.0
(22) Date of filing: 30.10.2018
(51) Int. Cl.: G01N 33/70

(54) **C5AR INHIBITOR REDUCTION OF URINARY SCD163**

(30) Priority: 31.10.2017 US 201762579716 P
(62) Divisional of application: 18873880.1
(71) Applicant: ChemoCentryx, Inc., Thousand Oaks, CA 91320 (US)
(72) Inventor: DENG, Jun, Cupertino, 95014 (US); SCHALL, Thomas J., Mountain View, 94043 (US); BEKKER, Petrus, Los Altos, 94022 (US)
(74) Representative: J A Kemp LLP

(57) **Abstract**

Provided herein are methods of treating ANCA-associated vasculitis (AAV) in individuals in need thereof comprising administering a complement component 5a receptor (C5aR) antagonist. Also provided herein are methods of treating a ANCA-associated vasculitis (AAV) with renal involvement in an individual in need thereof comprising administering a complement component 5a receptor (C5aR) antagonist to the individual if the individual exhibits an elevated urinary soluble CD163 (sCD163) to creatinine ratio compared to individuals without AAV. In some embodiments, the complement component 5a receptor (C5aR) antagonist is avacopan.

## Description

### CROSS-REFERENCES TO RELATED APPLICATIONS

This application is an application claiming benefit under 35 U.S.C. § 119(e) of U.S. Provisional Application No. 62/579,716 filed October 31, 2017, which is incorporated herein by reference in its entirety.

### STATEMENT AS TO RIGHTS TO INVENTIONS MADE UNDER FEDERALLY SPONSORED RESEARCH AND DEVELOPMENT

### REFERENCE TO A "SEQUENCE LISTING," A TABLE, OR A COMPUTER PROGRAM LISTING APPENDIX SUBMITTED ON A COMPACT DISK

### BACKGROUND

Anti-neutrophil cytoplasmic antibodies (ANCA) are a group of autoantibodies of the IgG type that react with the cytoplasmic constituents of neutrophils and monocytes. The presence of ANCA is with various idiopathic systemic vasculitis disorders (i.e., inflammation and weakening the blood vessel walls) and with other inflammatory disorders.

ANCA-associated vasculitis (AAV) is the pathologic condition in which ANCAs are detected in the serum, and which is very likely to be involved in rapidly progressive diseases including those with renal involvement.

Despite improved methods of identifying ANCAs and diagnosing associated diseases and disorders, there remains a need in the art to identify and develop compounds useful in the treatment of ANCA-associated vasculitis (AAV) and to identify biomarkers that accurately report on AAV disease status.

### BRIEF SUMMARY

The present disclosure is directed to, interalia, methods of treating ANCA-associated vasculitis (AAV) with renal involvement in an individual in need thereof comprising administering a complement component 5a receptor (C5aR) antagonist to the individual if the individual exhibits an elevated urinary soluble CD163 (sCD163) to creatinine ratio compared to individuals without AAV.

In another aspect, the present disclosure provides methods for treating ANCA-associated vasculitis (AAV) in an individual in need thereof comprising administering an effective amount of a complement component 5a receptor (C5aR) antagonist.

In some embodiment, the C5aR antagonist is a compound having the formula **(I),** or a pharmaceutically acceptable salt thereof, 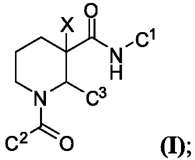 wherein
C¹ is phenyl optionally substituted with from 1 to 3 R¹ substituents;
C² is phenyl optionally substituted with from 1 to 3 R² substituents;
C³ is selected from the group consisting of C₃₋₈ cycloalkyl and phenyl, and each C³ is optionally substituted with from 1-3 R³ substituents;
each R¹ is independently selected from the group consisting of halogen, -CN, -R^{c}, -CO₂R^{a}, -CONR^{a}R^{b}, -C(O)R^{a}, -OC(O)NR^{a}R^{b}, -NR^{b}C(O)R^{a}, - NR^{b}C(O)₂R^{c}, -NR^{a}C(O)NR^{a}R^{b}, -NR^{a}R^{b}, -OR^{a}, and -S(O)₂NR^{a}R^{b}; wherein each R^{a} and R^{b} is independently selected from hydrogen, C₁₋₈ alkyl, and C₁₋₈ haloalkyl, or when attached to the same nitrogen atom can be combined with the nitrogen atom to form a five or six-membered ring having from 0 to 2 additional heteroatoms as ring members selected from N, O or S;
   each R^{c} is independently selected from the group consisting of C₁₋₈ alkyl, C₁₋₈ haloalkyl, C₃₋₆ cycloalkyl, heterocycloalkyl, aryl and heteroaryl, and wherein the aliphatic and cyclic portions of R^{a}, R^{b} and R^{c} are optionally further substituted with from one to three halogen, hydroxy, methyl, amino, alkylamino and dialkylamino groups; and optionally when two R¹ substituents are on adjacent atoms, are combined to form a fused five or six-membered carbocyclic ring;
each R² is independently selected from the group consisting of
   halogen, -CN, -R^{f}, -CO₂R^{d}, -CONR^{d}R^{e}, -C(O)R^{d}, -OC(O)NR^{d}R^{e}, -NR^{e}C(O)R^{d}, - NR^{e}C(O)₂R^{f}, -NR^{d}C(O)NR^{d}R^{e}, -NR^{d}C(O)NR^{d}R^{e}, -NR^{d}R^{e}, -OR^{d}, and -S(O)_{z}NR^{d}R^{e}; wherein each R^{d} and R^{e} is independently selected from hydrogen, C₁₋₈ alkyl, and C₁₋₈ haloalkyl, or when attached to the same nitrogen atom can be combined with the nitrogen atom to form a five or six-membered ring having from 0 to 2 additional heteroatoms as ring members
   selected from N, O or S; each R^{f} is independently selected from the group consisting of C₁₋₈ alkyl, C₁₋₈ haloalkyl, C₃₋₆ cycloalkyl, heterocycloalkyl, aryl and heteroaryl, and wherein the aliphatic and cyclic portions of R^{d}, R^{e} and R^{f} are optionally further substituted with from one to three halogen, hydroxy, methyl, amino, alkylamino and dialkylamino groups;
each R³ is independently selected from the group consisting of
   halogen, -CN, -Rⁱ, -CO₂R^{g}, -CONR^{g}R^{h}, -C(O)R^{g}, -OC(O)NR^{g}R^{h}, -NR^{h}C(O)R^{g}, - NR^{h}C(O)₂Rⁱ, -NR^{g}C(O)NR^{g}R^{h}, -NR^{g}R^{h}, -OR^{g}, -S(O)₂NR^{g}R^{h}, -X⁴-R^{j}, -X⁴-NR^{g}R^{h}, -X⁴-CONR ^{g}R^{h}, -X⁴-NR^{h}C(O)R^{g}, -NHR^{j} and -NHCH₂R^{j}, wherein X⁴ is a C₁₋₄ alkylene; each R^{g} and R^{h} is independently selected from hydrogen, C₁₋₈ alkyl, C₃₋₆ cycloalkyl and C₁₋₈ haloalkyl, or when attached to the same nitrogen atom can be combined with the nitrogen atom to form a five or six-membered ring having from 0 to 2 additional heteroatoms as ring members selected from N, O or S and is optionally substituted with one or two oxo; each Rⁱ is independently selected from the group consisting of C₁₋₈ alkyl, C₁₋₈ haloalkyl, C₃₋₆ cycloalkyl, heterocycloalkyl, aryl and heteroaryl; and each R^{j} is selected from the group consisting of C₃₋₆ cycloalkyl,
   pyrrolinyl, piperidinyl, morpholinyl, tetrahydrofuranyl, and tetrahydropyranyl, and wherein the aliphatic and cyclic portions of R^{g}, R^{h}, Rⁱ and R^{j} are optionally further substituted with from one to three halogen, methyl, CF₃, hydroxy, amino, alkylamino and dialkylamino groups; and
X is hydrogen or CH₃.

In some embodiments, the C5aR antagonist is a Avacopan, having the formula:

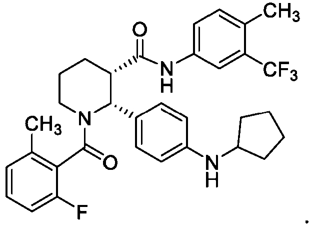

### BRIEF DESCRIPTION OF THE DRAWINGS

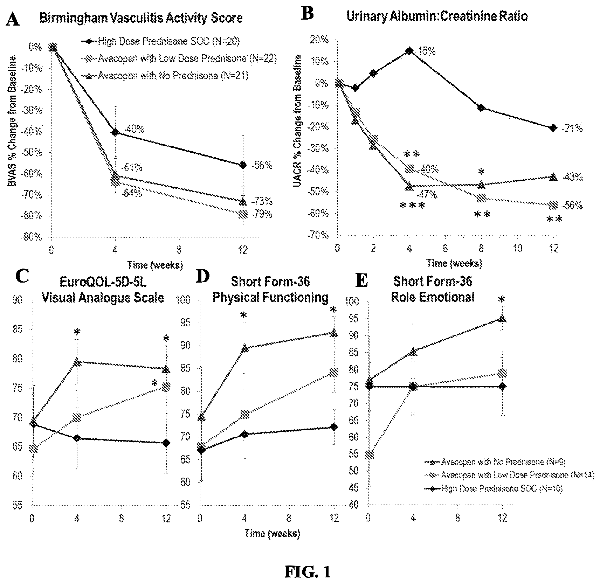

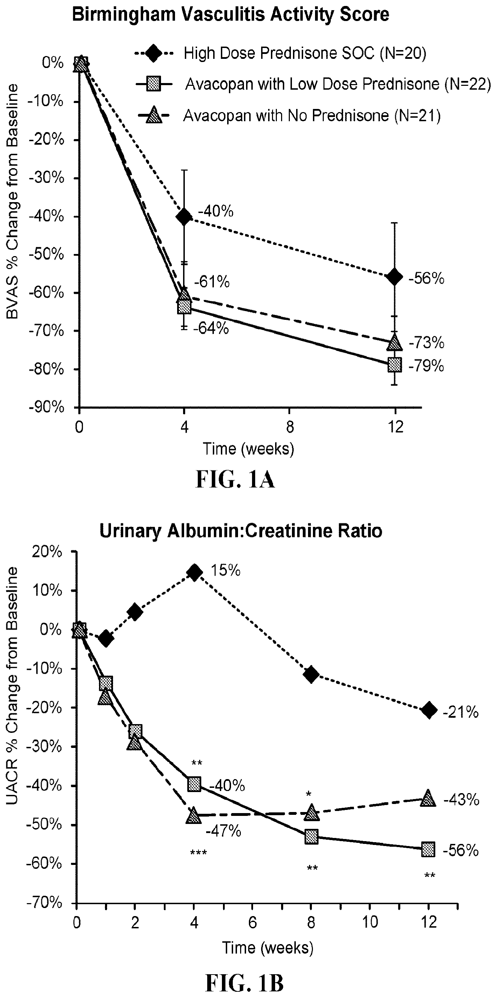

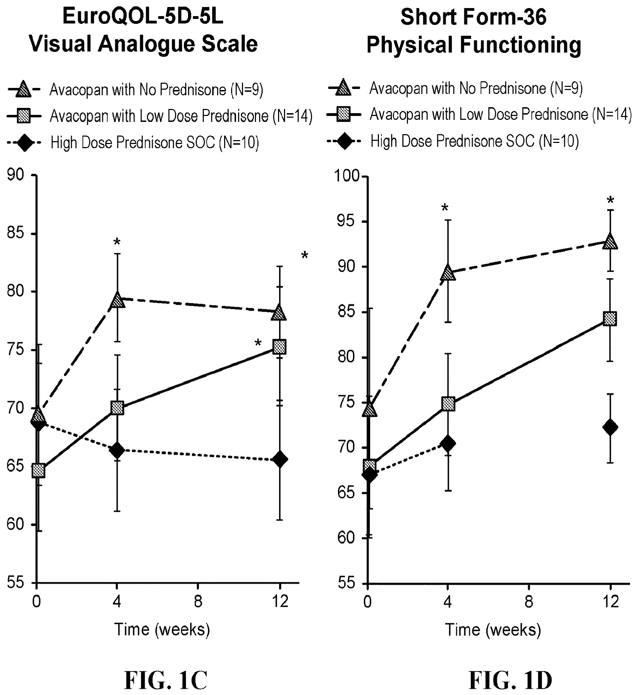

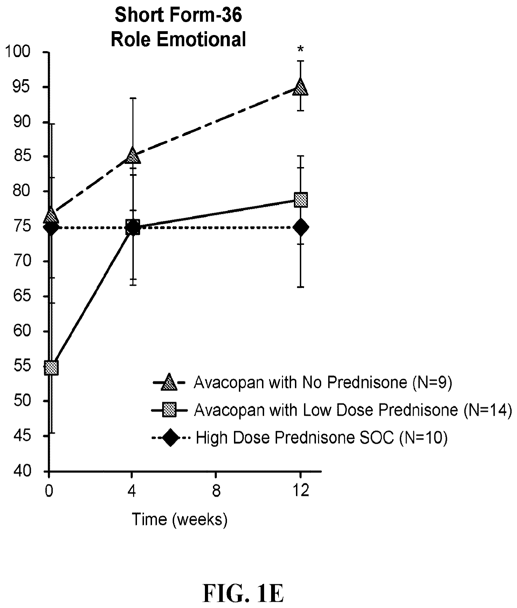

**FIG. 1A-E**illustrates the rapid decrease in diseases activity and significant improvement in health-related quality of life in patients treated with Avacopan. Panel A plots the Birmingham Vasculitis Activity Score; Panel B plots the Urinary Albumin: creatinine Ratio; Panel C plots the Health-related quality-of-life measurement EQ-5D-5L visual analog scale, expressed as mean +/- SEM over the treatment period; Panel D and Panel E plot the health-related quality-of-life measurement Medical Outcomes Study SF-36 version 2 Physical Functioning and Role Emotional components, expressed as mean +/- SEM over the treatment period.
**FIG. 2A-B**illustrates urinary sCD163/creatinine ratios at baseline. **FIG. 2A** shows levels of urinary sCD163/creatinine ratios in patients with anti-MPO and anti-PR3 autoantibodies; **FIG. 2B** shows the levels of urnary sCD163/creatinine ratios in patients randomized to three different treatment groups.
**FIG.** 3 illustrates the rapid reduction of urinary sCD163/creatinine ratio in patients treated with Avacopan. As shown in the graph, *p<0.05; **p<0.01; ***p<0.001; # p=0.076 is compared to baseline in each group.
**FIG. 4** illustrates the strong positive correction between urine sCD163/Cr and UACR. P<0.0001 for correlation; data from all subject and time points are included in the analysis.
**FIG. 5** illustrates the strong positive correction between urine sCD163/Cr and MCP-1/Cr. P<0.0001 for correlation; data from all subject and time points are included in the analysis.
**FIG. 6** illustrates the lack of correction between urine sCD163/Cr and eGFR. P=0.655 for correlation; data from all subject and time points are included in the analysis.
**FIG. 7A-C** illustrates the changes from baseline urine sCD163/Cr, eGFR, UACR, and MCP-1/Cr over time in all subjects. **FIG. 7A** plots eGFR and sCD163/Cr over time; **FIG. 7B** plots sCD163/Cr and UACR over time; **FIG. 7C** plots sCD163/Cr and MCP-1/Cr over time.

### DETAILED DESCRIPTION OF THE INVENTION

### GENERAL

The present disclosure provides methods of treating ANCA-associated vasculitis (AAV). In particular, the present disclosure demonstrates that avacopan effectively treats AAV in human subjects.

The present disclosure also demonstrates that in ANCA-associated vasculitis (AAV) patients receiving Avacopan, urine sCD163 decreased within one week, whereas in prednisone only treated AAV patients, urine sCD163 decreased much later (by week 8). Thus, treatment with Avacopan is also associated with a surprisingly rapid improvement of kidney inflammation. Unexpectedly, patients demonstrated a strong positive temporal correlation between urine monocyte chemoattractant protein-1 (MCP-1)/creatinine (Cr) ratio and sCD163/Cr ratio, while other renal function parameters such as eGFR and urine albumin to creatinine ratio (UACR) did not provide the same temporal correlation. Collectively, the provided data demonstrates that the urine sCD163/Cr ratio positively correlates with kidney inflammation in AAV patients and further indicates that resolution of kidney inflammation precedes improvement of kidney function.

### ABBREVIATION AND DEFINITIONS

The term "alkyl", by itself or as part of another substituent, means, unless otherwise stated, a straight or branched chain hydrocarbon radical, having the number of carbon atoms designated (*i.e.* C₁₋₈ means one to eight carbons). Examples of alkyl groups include methyl, ethyl, n-propyl, isopropyl, n-butyl, t-butyl, isobutyl, sec-butyl, n-pentyl, n-hexyl, n-heptyl, n-octyl, and the like. The term "alkenyl" refers to an unsaturated alkyl group having one or more double bonds. Similarly, the term "alkynyl" refers to an unsaturated alkyl group having one or more triple bonds. Examples of such unsaturated alkyl groups include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "cycloalkyl" refers to hydrocarbon rings having the indicated number of ring atoms (*e.g.*, C₃₋₆cycloalkyl) and being fully saturated or having no more than one double bond between ring vertices. "Cycloalkyl" is also meant to refer to bicyclic and polycyclic hydrocarbon rings such as, for example, bicyclo[2.2.1]heptane, bicyclo[2.2.2]octane, etc. The term "heterocycloalkyl" refers to a cycloalkyl group that contain from one to five heteroatoms selected from N, O, and S, wherein the nitrogen and sulfur atoms are optionally oxidized, and the nitrogen atom(s) are optionally quaternized. The heterocycloalkyl may be a monocyclic, a bicyclic or a polycylic ring system. Non limiting examples of heterocycloalkyl groups include pyrrolidine, imidazolidine, pyrazolidine, butyrolactam, valerolactam, imidazolidinone, hydantoin, dioxolane, phthalimide, piperidine, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrhydrothiophene, quinuclidine, and the like. A heterocycloalkyl group can be attached to the remainder of the molecule through a ring carbon or a heteroatom.

The term "alkylene" by itself or as part of another substituent means a divalent radical derived from an alkane, as exemplified by -CH₂CH₂CH₂CH₂-. Typically, an alkyl (or alkylene) group will have from 1 to 24 carbon atoms, with those groups having 10 or fewer carbon atoms being preferred in the present disclosure. A "lower alkyl" or "lower alkylene" is a shorter chain alkyl or alkylene group, generally having four or fewer carbon atoms. Similarly, "alkenylene" and "alkynylene" refer to the unsaturated forms of "alkylene" having double or triple bonds, respectively.

The term "heteroalkyl," by itself or in combination with another term, means, unless otherwise stated, a stable straight or branched chain, or cyclic hydrocarbon radical, or combinations thereof, consisting of the stated number of carbon atoms and from one to three heteroatoms selected from the group consisting of O, N, Si and S, and wherein the nitrogen and sulfur atoms may optionally be oxidized and the nitrogen heteroatom may optionally be quaternized. The heteroatom(s) O, N and S may be placed at any interior position of the heteroalkyl group. The heteroatom Si may be placed at any position of the heteroalkyl group, including the position at which the alkyl group is attached to the remainder of the molecule. Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, and -CH=CH-N(CH₃)-CH₃. Up to two heteroatoms may be consecutive, such as, for example, -CH₂-NH-OCH₃ and -CH₂-O-Si(CH₃)₃. Similarly, the terms "heteroalkenyl" and "heteroalkynyl" by itself or in combination with another term, means, unless otherwise stated, an alkenyl group or alkynyl group, respectively, that contains the stated number of carbons and having from one to three heteroatoms selected from the group consisting of O, N, Si and S, and wherein the nitrogen and sulfur atoms may optionally be oxidized and the nitrogen heteroatom may optionally be quaternized. The heteroatom(s) O, N and S may be placed at any interior position of the heteroalkyl group.

The term "heteroalkylene" by itself or as part of another substituent means a divalent radical, saturated or unsaturated or polyunsaturated, derived from heteroalkyl, as exemplified by -CH₂-CH₂-S-CH₂CH₂- and -CH₂-S-CH₂-CH₂-NH-CH₂- , -O-CH₂-CH=CH-, -CH₂-CH=C(H)CH₂-O-CH₂- and -S-CH₂-C≡C-. For heteroalkylene groups, heteroatoms can also occupy either or both of the chain termini (e.g., alkyleneoxy, alkylenedioxy, alkyleneamino, alkylenediamino, and the like).

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively. Additionally, for dialkylamino groups, the alkyl portions can be the same or different and can also be combined to form a 3-7 membered ring with the nitrogen atom to which each is attached. Accordingly, a group represented as -NR^{a}R^{b} is meant to include piperidinyl, pyrrolidinyl, morpholinyl, azetidinyl and the like.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "C₁-₄ haloalkyl" is mean to include trifluoromethyl, 2,2,2-trifluoroethyl, 4-chlorobutyl, 3-bromopropyl, and the like.

The term "aryl" means, unless otherwise stated, a polyunsaturated, typically aromatic, hydrocarbon group which can be a single ring or multiple rings (up to three rings) which are fused together or linked covalently. The term "heteroaryl" refers to aryl groups (or rings) that contain from one to five heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl groups include phenyl, naphthyl and biphenyl, while non-limiting examples of heteroaryl groups include pyridyl, pyridazinyl, pyrazinyl, pyrimindinyl, triazinyl, quinolinyl, quinoxalinyl, quinazolinyl, cinnolinyl, phthalaziniyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridines, benzothiaxolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, thiadiazolyl, pyrrolyl, thiazolyl, furyl, thienyl and the like. Substituents for each of the above noted aryl and heteroaryl ring systems are selected from the group of acceptable substituents described below.

For brevity, the term "aryl" when used in combination with other terms (*e.g.,* aryloxy, arylthioxy, arylalkyl) includes both aryl and heteroaryl rings as defined above. Thus, the term "arylalkyl" is meant to include those radicals in which an aryl group is attached to an alkyl group (*e.g.,* benzyl, phenethyl, pyridylmethyl and the like).

The above terms (*e.g.,* "alkyl," "aryl" and "heteroaryl"), in some embodiments, will include both substituted and unsubstituted forms of the indicated radical. Preferred substituents for each type of radical are provided below. For brevity, the terms aryl and heteroaryl will refer to substituted or unsubstituted versions as provided below, while the term "alkyl" and related aliphatic radicals is meant to refer to unsubstituted version, unless indicated to be substituted.

Substituents for the alkyl radicals (including those groups often referred to as alkylene, alkenyl, alkynyl and cycloalkyl) can be a variety of groups selected from: -halogen, -OR', - NR'R", -SR', -SiR'R"R‴, -OC(O)R', -C(O)R', -CO₂R', -CONR'R", -OC(O)NR'R", - NR"C(O)R', -NR'-C(O)NR"R‴, -NR"C(O)₂R', -NH-C(NH₂)=NH, -NR'C(NH₂)=NH, -NH-C(NH₂)=NR', -S(O)R', -S(O)₂R', -S(O)₂NR'R", -NR'S(O)₂R", -CN and -NO₂ in a number ranging from zero to (2 m'+1), where m' is the total number of carbon atoms in such radical. R', R" and R‴ each independently refer to hydrogen, unsubstituted C₁-₈ alkyl, unsubstituted heteroalkyl, unsubstituted aryl, aryl substituted with 1-3 halogens, unsubstituted C₁₋₈ alkyl, C₁₋₈ alkoxy or C₁₋₈ thioalkoxy groups, or unsubstituted aryl-C₁-₄ alkyl groups. When R' and R" are attached to the same nitrogen atom, they can be combined with the nitrogen atom to form a 3-, 4-, 5-, 6-, or 7-membered ring. For example, -NR'R" is meant to include 1-pyrrolidinyl and 4-morpholinyl. The term "acyl" as used by itself or as part of another group refers to an alkyl radical wherein two substitutents on the carbon that is closest to the point of attachment for the radical is replaced with the substitutent =O (e.g., -C(O)CH₃, -C(O)CH₂CH₂OR' and the like).

Similarly, substituents for the aryl and heteroaryl groups are varied and are generally selected from: -halogen, -OR', -OC(O)R', -NR'R", -SR', -R', -CN, -NO₂, - CO₂R', -CONR'R", -C(O)R', -OC(O)NR'R", -NR"C(O)R', -NR"C(O)₂R', ,-NR'-C(O)NR"R"', -NH-C(NH₂)=NH, -NR'C(NH₂)=NH, -NH-C(NH₂)=NR', -S(O)R', - S(O)₂R', -S(O)₂NR'R", -NR'S(O)₂R", -N₃, perfluoro(C₁-C₄)alkoxy, and perfluoro(C₁-C₄)alkyl, in a number ranging from zero to the total number of open valences on the aromatic ring system; and where R', R" and R‴ are independently selected from hydrogen, C₁₋₈ alkyl, C₃₋₆ cycloalkyl, C₂₋₈ alkenyl, C₂₋₈ alkynyl, unsubstituted aryl and heteroaryl, (unsubstituted aryl)-C₁-₄ alkyl, and unsubstituted aryloxy-C₁-₄ alkyl. Other suitable substituents include each of the above aryl substituents attached to a ring atom by an alkylene tether of from 1-4 carbon atoms.

Two of the substituents on adjacent atoms of the aryl or heteroaryl ring may optionally be replaced with a substituent of the formula -T-C(O)-(CH₂)_{q}-U-, wherein T and U are independently -NH-, -O-, -CH₂- or a single bond, and q is an integer of from 0 to 2.

Alternatively, two of the substituents on adjacent atoms of the aryl or heteroaryl ring may optionally be replaced with a substituent of the formula -A-(CH₂)ᵣ-B-, wherein A and B are independently -CH₂-, -O-, -NH-, -S-, -S(O)-, -S(O)₂-, -S(O)₂NR'- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR'-, -S-, -S(O)-, - S(O)₂-, or -S(O)₂NR'-. The substituent R' in -NR'- and -S(O)₂NR'- is selected from hydrogen or unsubstituted C₁-₆ alkyl.

As used herein, the term "heteroatom" is meant to include oxygen (O), nitrogen (N), sulfur (S) and silicon (Si).

As used herein, the term "treating" or "treatment" encompasses both disease-modifying treatment and symptomatic treatment, either of which may be prophylactic (i.e., before the onset of symptoms, in order to prevent, delay or reduce the severity of symptoms) or therapeutic (i.e., after the onset of symptoms, in order to reduce the severity and/or duration of symptoms).

The term "pharmaceutically acceptable salts" is meant to include salts of the active compounds which are prepared with relatively nontoxic acids or bases, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Examples of salts derived from pharmaceutically-acceptable inorganic bases include aluminum, ammonium, calcium, copper, ferric, ferrous, lithium, magnesium, manganic, manganous, potassium, sodium, zinc and the like. Salts derived from pharmaceutically-acceptable organic bases include salts of primary, secondary and tertiary amines, including substituted amines, cyclic amines, naturally-occuring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. When compounds of the present disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S.M., et al, "Pharmaceutical Salts", Journal of Pharmaceutical Science, 1977, 66, 1-19). Certain specific compounds of the present disclosure contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present disclosure.

The compounds described in the Embodiments below can be obtained according to methods described in WO 2010/075257, WO 2011/163640 and WO 2016/053890.

### EMBODIMENTS

The present disclosure is directed to, *inter alia,* a method of treating ANCA-associated vasculitis (AAV) with renal involvement in an individual in need thereof comprising administering a complement component 5a receptor (C5aR) antagonist to the individual if the individual exhibits an elevated urinary soluble CD163 (sCD163) to creatinine ratio compared to individuals without AAV.

In some aspects, provided herein are methods for assessing the effectiveness of treatment with a complement component 5a receptor (C5aR) antagonist in an individual diagnosed with or thought to have ANCA-associated vasculitis (AAV), the method comprising:
measuring the soluble CD163 (sCD163) to creatinine ratio in a urinary sample from the individual prior and subsequent to administration of the C5aR antagonist, wherein treatment is effective if the concentration of urinary sCD163 decreases subsequent to administration.

In some embodiments, an effective treatment decreases the sCD163 to creatinine ratio in a urinary sample from the individual subsequent to administration of the C5aR antagonist by at least 20, 30, 40, 50, 60, 70, 80% or more as compared to the sCD163 to creatinine ratio in a urinary sample from the individual prior to administration of the C5aR antagonist. In some embodiments, an effective treatment decreases the sCD163 to creatinine ratio in a urinary sample from the individual subsequent to administration of the C5aR antagonist by at least 40% as compared to the sCD163 to creatinine ratio in a urinary sample from the individual prior to administration of the C5aR antagonist. In some embodiments, an effective treatment decreases the sCD163 to creatinine ratio in a urinary sample from the individual subsequent to administration of the C5aR antagonist by at least 50% as compared to the sCD163 to creatinine ratio in a urinary sample from the individual prior to administration of the C5aR antagonist. In some embodiments, an effective treatment decreases the sCD163 to creatinine ratio in a urinary sample from the individual subsequent to administration of the C5aR antagonist by at least 60% as compared to the sCD163 to creatinine ratio in a urinary sample from the individual prior to administration of the C5aR antagonist.

In some embodiments, the urinary sCD163 to creatinine ratio is measured relative to the ratio of albumin to creatinine in a urinary sample from the individual.

In some embodiments, the urinary sCD163 to creatinine ratio is measured relative to the ratio of monocyte chemoattractant protein-1 (MCP-1) to creatinine in a urinary sample from the individual.

The methods of treating ANCA-associated vasculitis (AAV) described herein provide a reduction in the concentration of urinary soluble CD163 (sCD163), and the amount will vary depending on the starting amount of sCD163 and the disease state of the individual. In some embodiments, the concentration of sCD163 in a urinary sample from the individual after 8 days of administering a C5aR antagonist is reduced by at least 10, 15, 20, 25, 30, 35, 40 45, 50 %, or more. In some embodiments, the concentration of sCD163 in a urinary sample from the individual after 8 days of administering a C5aR antagonist is reduced by at least 20 %. In some embodiments, the concentration of sCD163 in a urinary sample from the individual after 8 days of administering a C5aR antagonist is reduced by at least 25 %. In some embodiments, the concentration of sCD163 in a urinary sample from the individual after 8 days of administering a C5aR antagonist is reduced by at least 30 %.

In some aspects, provided herein are methods for reducing the concentration of urinary soluble CD163 (sCD163) in an individual diagnosed with or thought to have ANCA-associated vasculitis (AAV), the method comprising:
administering a complement component 5a receptor (C5aR) antagonist to the individual, wherein administration of the C5aR antagonist decreases the concentration of urinary sCD163 in a urinary sample provided by the individual.

The relative reduction in the concentration of urinary soluble CD163 (sCD163) will vary depending on the starting amount and the disease state of the individual. In some embodiments, the concentration of sCD163 in a urinary sample from the individual after 8 days of administering a C5aR antagonist is reduced by at least 10, 15, 20, 25, 30, 35, 40 45, 50 %, or more. In some embodiments, the concentration of sCD163 in a urinary sample from the individual after 8 days of administering a C5aR antagonist is reduced by at least 20 %. In some embodiments, the concentration of sCD163 in a urinary sample from the individual after 8 days of administering a C5aR antagonist is reduced by at least 25 %. In some embodiments, the concentration of sCD163 in a urinary sample from the individual after 8 days of administering a C5aR antagonist is reduced by at least 30 %.

In some aspects, provided herein are methods for treating ANCA-associated vasculitis (AAV) in an individual in need thereof comprising administering an effective amount of a complement component 5a receptor (C5aR) antagonist, thereby treating ANCA-associated vasculitis. Indeed patients administered a complement component 5a receptor (C5aR) antagonist (avacopan) demonstrated a surprising quick response to treatment. In some embodiments, the individual has ANCA-associate vasculitis with rental involvement.

In some embodiments, treatment with the complement component 5a receptor (C5aR) antagonist avacopan reduces the urinary albumin to creatinine ratio of an individual by at least 30% after 4 weeks of treatment as compared to the urinary albumin to creatinine ratio of the individual prior to treatment. In some embodiments, treatment with the complement component 5a receptor (C5aR) antagonist avacopan reduces the urinary albumin to creatinine ratio of an individual by at least 35% after 4 weeks of treatment as compared to the urinary albumin to creatinine ratio of the individual prior to treatment. In some embodiments, treatment with the complement component 5a receptor (C5aR) antagonist avacopan reduces the urinary albumin to creatinine ratio of an individual by at least 40% after 4 weeks of treatment as compared to the urinary albumin to creatinine ratio of the individual prior to treatment. In some embodiments, treatment with the complement component 5a receptor (C5aR) antagonist avacopan reduces the urinary albumin to creatinine ratio of the individual by at least 45% after 4 weeks of treatment as compared to the urinary albumin to creatinine ratio of the individual prior to treatment.

In some embodiments, treatment with the complement component 5a receptor (C5aR) antagonist avacopan reduces the Birmingham vasculitis activity score of an individual by at least 50% after 4 weeks of treatment as compared to the score of the individual prior to treatment. In some embodiments, treatment with the complement component 5a receptor (C5aR) antagonist avacopan reduces the Birmingham vasculitis activity score of the individual by at least 60% after 4 weeks of treatment as compared to the score of the individual prior to treatment..

In some embodiments, the C5aR antagonist is a compound of Formula (I), or a pharmaceutically acceptable salt thereof, 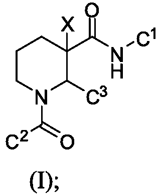 wherein
C¹ is phenyl optionally substituted with from 1 to 3 R¹ substituents;
C² is phenyl optionally substituted with from 1 to 3 R² substituents;
C³ is selected from the group consisting of C₃₋₈ cycloalkyl and phenyl, and each C³ is optionally substituted with from 1-3 R³ substituents;
each R¹ is independently selected from the group consisting of
   halogen, -CN, -R^{c}, -CO₂R^{a}, -CONR^{a}R^{b}, -C(O)R^{a}, -OC(O)NR^{a}R^{b}, -NR^{b}C(O)R^{a}, - NR^{b}C(O)₂R^{c}, -NR^{a}C(O)NR^{a}R^{b}, -NR^{a}R^{b}, -OR^{a}, and -S(O)₂NR^{a}R^{b}; wherein each R^{a} and R^{b} is independently selected from hydrogen, C₁₋₈ alkyl, and C₁₋₈ haloalkyl, or when attached to the same nitrogen atom can be combined with the nitrogen atom to form a five or six-membered ring having from 0 to 2 additional heteroatoms as ring members selected from N, O or S;
   each R^{c} is independently selected from the group consisting of C₁₋₈ alkyl, C₁₋₈ haloalkyl, C₃₋₆ cycloalkyl, heterocycloalkyl, aryl and heteroaryl, and wherein the aliphatic and cyclic portions of R^{a}, R^{b} and R^{c} are optionally further substituted with from one to three halogen, hydroxy, methyl, amino, alkylamino and dialkylamino groups; and optionally when two R¹ substituents are on adjacent atoms, are combined to form a fused five or six-membered carbocyclic ring;
each R² is independently selected from the group consisting of
   halogen, -CN, -R^{f}, -CO₂R^{d}, -CONR^{d}R^{e}, -C(O)R^{d}, -OC(O)NR^{d}R^{e}, -NR^{e}C(O)R^{d}, - NR^{e}C(O)₂R^{f}; -NR^{d}C(O)NR^{d}R^{e}, -NR^{d}C(O)NR^{d}R^{e}, -NR^{d}R^{e}, -OR^{d}, and -S(O)₂NR^{d}R^{e}; wherein each R^{d} and R^{e} is independently selected from hydrogen, C₁₋₈ alkyl, and C₁₋₈ haloalkyl, or when attached to the same nitrogen atom can be combined with the nitrogen atom to form a five or six-membered ring having from 0 to 2 additional heteroatoms as ring members selected from N, O or S; each R^{f} is independently selected from the group consisting of C₁₋₈ alkyl, C₁₋₈ haloalkyl, C₃₋₆ cycloalkyl, heterocycloalkyl, aryl and heteroaryl, and wherein the aliphatic and cyclic portions of R^{d}, R^{e} and R^{f} are optionally further substituted with from one to three halogen, hydroxy, methyl, amino, alkylamino and dialkylamino groups;
each R³ is independently selected from the group consisting of
   halogen, -CN, -Rⁱ, -CO₂R^{g}, -CONR^{g}R^{h}, -C(O)R^{g}, -OC(O)NR^{g}R^{h}, -NR^{h}C(O)R^{g}, - NR^{h}C(O)₂Rⁱ, -NR^{g}C(O)N2R^{g}R^{h}, -NR^{g}R^{h}, -OR^{g}, -S(O)₂NR^{g}R^{h}, -X⁴-R^{j}, -X⁴-NR^{g}R^{h}, -X⁴-CONR ^{g}R^{h}, -X⁴-NR^{h}C(O)R^{g}, -NHR^{j} and -NHCH₂R^{j}, wherein X⁴ is a C₁₋₄ alkylene; each R^{g} and R^{h} is independently selected from hydrogen, C₁₋₈ alkyl, C₃₋₆ cycloalkyl and C₁₋₈ haloalkyl, or when attached to the same nitrogen atom can be combined with the nitrogen atom to form a five or six-membered ring having from 0 to 2 additional heteroatoms as ring members selected from N, O or S and is optionally substituted with one or two oxo; each Rⁱ is independently selected from the group consisting of C₁₋₈ alkyl, C₁₋₈ haloalkyl, C₃₋₆ cycloalkyl, heterocycloalkyl, aryl and heteroaryl; and each R^{j} is selected from the group consisting of C₃₋₆ cycloalkyl, pyrrolinyl, piperidinyl, morpholinyl, tetrahydrofuranyl, and tetrahydropyranyl, and wherein the aliphatic and cyclic portions of R^{g}, R^{h}, Rⁱ and R^{j} are optionally further substituted with from one to three halogen, methyl, CF₃, hydroxy, amino, alkylamino and dialkylamino groups; and
X is hydrogen or CH₃.

In some embodiments, the C5aR antagonist has the formula (Ia):

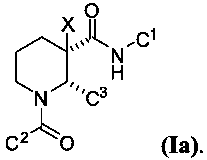

In some embodiments, the C5aR antagonist has the formula (Ib): 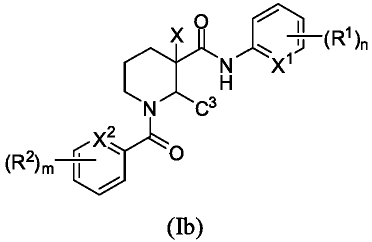 wherein
X¹ is selected from the group consisting of CH and CR¹;
the subscript n is an integer of from 0 to 2;
X² is selected from the group consisting of CH and CR²; and
the subscript m is an integer of from 0 to 2.

In some embodiments, the C5aR antagonist has the formula (Ic): 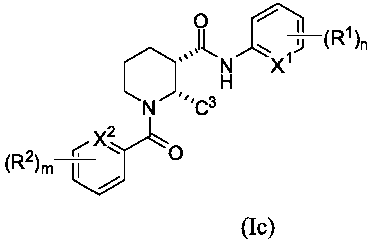 wherein
X¹ is selected from the group consisting of CH and CR¹;
the subscript n is an integer of from 0 to 2;
X² is selected from the group consisting of CH and CR²; and
the subscript m is an integer of from 0 to 2.

In some embodiments, the C5aR antagonist has the formula (Id): 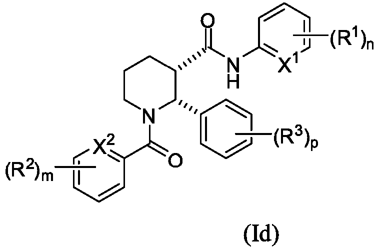 wherein
the subscript p is an integer of from 0 to 3;
X¹ is selected from the group consisting of CH and CR¹;
the subscript n is an integer of from 0 to 2;
X² is selected from the group consisting of CH and CR²; and
the subscript m is an integer of from 0 to 2.

In some embodiments, the compound has the formula (Ie): 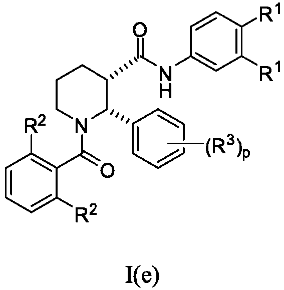 wherein p is 0, 1 or 2.

In some embodiments, the C5aR antagonist is Avacopan, having the formula 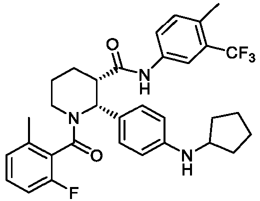 or a pharmaceutically acceptable salt thereof.

Certain compounds of the present disclosure can exist in unsolvated forms as well as solvated forms, including hydrated forms. In general, the solvated forms are equivalent to unsolvated forms and are intended to be encompassed within the scope of the present disclosure. Certain compounds of the present disclosure may exist in multiple crystalline or amorphous forms. In general, all physical forms are equivalent for the uses contemplated by the present disclosure and are intended to be within the scope of the present disclosure.

Certain compounds of the present disclosure possess asymmetric carbon atoms (optical centers) or double bonds; the racemates, diastereomers, geometric isomers, regioisomers and individual isomers (*e.g.,* separate enantiomers) are all intended to be encompassed within the scope of the present disclosure. The compounds of the present disclosure may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present disclosure, whether radioactive or not, are intended to be encompassed within the scope of the present disclosure.

The compounds disclosed herein are also meant to encompass all pharmaceutically acceptable compounds of Formulas (I), (Ia), (Ib), (Ic), (Id), (Ie) and Avacopan being isotopically-labeled by having one or more atoms replaced by an atom having a different atomic mass or mass number. Examples of isotopes that can be incorporated into the disclosed compounds include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²O, ³⁵S, ¹⁸F, ³⁶Cl, ¹²³I, and ¹²⁵I, respectively. These radiolabeled compounds could be useful to help determine or measure the effectiveness of the compounds, by characterizing, for example, the site or mode of action, or binding affinity to pharmacologically important site of action. Certain isotopically-labeled compounds of Formulas (I), (Ia), (Ib), (Ic), (Id), (Ie) and Avacopan for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Substitution with heavier isotopes such as deuterium, *i.e.* ²H, may afford certain therapeutic advantages resulting from greater metabolic stability. For example, *in vivo* half-life may increase or dosage requirements may be reduced. Thus, heavier isotopes may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of Formulas (I), (Ia), (Ib), (Ic), (Id), (Ie) and Avacopan can generally be prepared by conventional techniques known to those skilled in the art or by processes analogous to those described in the Examples as set out below using an appropriate isotopically-labeled reagent in place of the non-labeled reagent previously employed.

The methods, compositions, kits and articles of manufacture provided herein use or include compounds (e.g., (I), (Ia), (Ib), (Ic), (Id), (Ie) and Avacopan) or pharmaceutically acceptable salts, prodrugs, or solvates thereof, in which from 1 to n hydrogen atoms attached to a carbon atom may be replaced by a deuterium atom or D, in which n is the number of hydrogen atoms in the molecule. As known in the art, the deuterium atom is a non-radioactive isotope of the hydrogen atom. Such compounds may increase resistance to metabolism, and thus may be useful for increasing the half-life of compounds or pharmaceutically acceptable salts, prodrugs, or solvates thereof, when administered to a mammal. See, e.g., Foster, "Deuterium Isotope Effects in Studies of Drug Metabolism", Trends Pharmacol. Sci., 5(12):524-527 (1984). Such compounds are synthesized by means well known in the art, for example by employing starting materials in which one or more hydrogen atoms have been replaced by deuterium.

Treatment methods provided herein include, in general, administration to a patient an effective amount of the compounds provided herein. Suitable patients include those patients suffering from or susceptible to anti-neutrophil cytoplasmic antibody (ANCA) -associated vasculitis (AAV).

In general, treatment methods provided herein comprise administering to a patient an effective amount of a compound provided herein. In a preferred embodiment, the compound(s) of the disclosure are preferably administered to a patient (*e.g.,* a human) orally, topically. In another embodiment, the compound(s) of the disclosure are administered to a patient (*e.g.,* a human) systemically (intravenously or subcutaneously). The effective amount may be an amount sufficient to modulate C5a receptor activity and/or an amount sufficient to reduce or alleviate the symptoms presented by the patient. Preferably, the amount administered is sufficient to yield a plasma concentration of the compound (or its active metabolite, if the compound is a pro-drug) high enough to detectably inhibit white blood cell (*e.g.,* neutrophil) chemotaxis *in vitro.* Treatment regimens may vary depending on the compound used and the particular condition to be treated; for treatment of most disorders, a frequency of administration of 4 times daily or less is preferred. In general, a dosage regimen of 2 times daily is more preferred, with once a day dosing particularly preferred. It will be understood, however, that the specific dose level and treatment regimen for any particular patient will depend upon a variety of factors including the activity of the specific compound employed, the age, body weight, general health, sex, diet, time of administration, route of administration, rate of excretion, drug combination (*i.e.,* other drugs being administered to the patient) and the severity of the particular disease undergoing therapy, as well as the judgment of the prescribing medical practitioner. In general, the use of the minimum dose sufficient to provide effective therapy is preferred. Patients may generally be monitored for therapeutic effectiveness using medical or veterinary criteria suitable for the condition being treated or prevented.

Dosage levels of the order of from about 0.1 mg to about 140 mg per kilogram of body weight per day are useful in the treatment or preventions of conditions involving pathogenic C5a activity (about 0.5 mg to about 7 g per human patient per day). The amount of active ingredient that may be combined with the carrier materials to produce a single dosage form will vary depending upon the host treated and the particular mode of administration. Dosage unit forms will generally contain between from about 1 mg to about 500 mg of an active ingredient. In some embodiments, dosage unit form are 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, or 100 mg of drug substance. For compounds administered orally, transdermally, intravaneously, or subcutaneously, it is preferred that sufficient amount of the compound be administered to achieve a serum concentration of 5 ng (nanograms)/mL-10 µg (micrograms)/mL serum, more preferably sufficient compound to achieve a serum concentration of 20 ng-1 µg/ml serum should be administered, most preferably sufficient compound to achieve a serum concentration of 50 ng/ml-200 ng/ml serum should be administered. For direct injection into the synovium (for the treatment of arthritis) sufficient compounds should be administered to achieve a local concentration of approximately 1 micromolar.

In some embodiments, the amount of the C5aR antagonist administered is 30 mg. In some embodiments, the total daily dose of the C5aR antagonist is 60 mg. In some embodiments, the C5aR antagonist is administered orally. In some embodiments, 30 mg of the C5aR antagonist is administered twice daily via oral administration.

Frequency of dosage may also vary depending on the compound used and the particular disease treated. However, for treatment of most disorders, a dosage regimen of 4 times daily, three times daily, or less is preferred, with a dosage regimen of once daily or 2 times daily being particularly preferred. It will be understood, however, that the specific dose level for any particular patient will depend upon a variety of factors including the activity of the specific compound employed, the age, body weight, general health, sex, diet, time of administration, route of administration, and rate of excretion, drug combination (*i.e.,* other drugs being administered to the patient), the severity of the particular disease undergoing therapy, and other factors, including the judgment of the prescribing medical practitioner.

### PHARMACEUTICAL COMPOSITIONS

The compounds provided herein can be administered as compositions which will typically contain a pharmaceutical carrier or diluent.

The term "composition" as used herein is intended to encompass a product comprising the specified ingredients in the specified amounts, as well as any product which results, directly or indirectly, from combination of the specified ingredients in the specified amounts.

In some embodiments, the pharmaceutical composition further comprises one or more additional therapeutic agents.

The pharmaceutical compositions for the administration of the compounds of this disclosure may conveniently be presented in unit dosage form and may be prepared by any of the methods well known in the art of pharmacy and drug delivery. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases.

The pharmaceutical compositions containing the active ingredient may be in a form suitable for oral use, for example, as tablets, troches, lozenges, aqueous or oily suspensions, dispersible powders or granules, emulsions and self-emulsifications as described in U.S. Patent Application 2002-0012680, hard or soft capsules, syrups, elixirs, solutions, buccal patch, oral gel, chewing gum, chewable tablets, effervescent powder and effervescent tablets. Compositions intended for oral use may be prepared according to any method known to the art for the manufacture of pharmaceutical compositions and such compositions may contain one or more agents selected from the group consisting of sweetening agents, flavoring agents, coloring agents, antioxidants and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as cellulose, silicon dioxide, aluminum oxide, calcium carbonate, sodium carbonate, glucose, mannitol, sorbitol, lactose, calcium phosphate or sodium phosphate; granulating and disintegrating agents, for example, corn starch, or alginic acid; binding agents, for example PVP, cellulose, PEG, starch, gelatin or acacia, and lubricating agents, for example magnesium stearate, stearic acid or talc. The tablets may be uncoated or they may be coated, enterically or otherwise, by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Pat. Nos. 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, polyethylene glycol (PEG) of various average sizes (e.g., PEG400, PEG4000) and certain surfactants such as cremophor or solutol, or as soft gelatin capsules wherein the active ingredient is mixed with water or an oil medium, for example peanut oil, liquid paraffin, or olive oil. Additionally, emulsions can be prepared with a non-water miscible ingredient such as oils and stabilized with surfactants such as mono- or di-glycerides, PEG esters and the like.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxy-ethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the disclosure may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. Oral solutions can be prepared in combination with, for example, cyclodextrin, PEG and surfactants.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of the present disclosure may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter and polyethylene glycols. Additionally, the compounds can be administered via ocular delivery by means of solutions or ointments. Still further, transdermal delivery of the subject compounds can be accomplished by means of iontophoretic patches and the like. For topical use, creams, ointments, jellies, solutions or suspensions, etc., containing the compounds of the present disclosure are employed. As used herein, topical application is also meant to include the use of mouth washes and gargles.

The compounds of this disclosure may also be coupled a carrier that is a suitable polymers as targetable drug carriers. Such polymers can include polyvinylpyrrolidone, pyran copolymer, polyhydroxy-propyl-methacrylamide-phenol, polyhydroxyethyl-aspartamide-phenol, or polyethyleneoxide-polylysine substituted with palmitoyl residues. Furthermore, the compounds of the disclosure may be coupled to a carrier that is a class of biodegradable polymers useful in achieving controlled release of a drug, for example polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels. Polymers and semipermeable polymer matrices may be formed into shaped articles, such as valves, stents, tubing, prostheses and the like. In one embodiment of the disclosure, the compound of the disclosure is coupled to a polymer or semipermeable polymer matrix that is formed as a stent or stent-graft device.

### KITS AND PACKAGES

The terms "kit" and "pharmaceutical kit" refer to a commercial kit or package comprising, in one or more suitable containers, one or more pharmaceutical compositions and instructions for their use. In one embodiment, kits comprising a compound of Formula (I), (Ia), (Ib), (Ic), (Id) or (Ie), or Avacopan, or a pharmaceutically acceptable salt thereof, and instructions for its administration are provided. In one embodiment, kits comprising a compound of Formula (I), (Ia), (Ib), (Ic), (Id) or (Ie), or Avacopan, or a pharmaceutically acceptable salt thereof, in combination with one or more (e.g., one, two, three, one or two, or one to three) additional therapeutic agents and instructions for their administration are provided.

In one embodiment, the compounds of this disclosure are formulated into administration units which are packaged in a single packaging. The single packaging encompasses but is not limited to a bottle, a child-resistant bottle, an ampoule, and a tube. In one embodiment, the compounds of this disclosure and optionally additional therapeutic agents, are formulated into administration units and every single administration unit is individually packaged in a single packaging. Such individually packaged units may contain the pharmaceutical composition in any form including but not limited to liquid form, solid form, powder form, granulate form, an effervescent powder or tablet, hard or soft capsules, emulsions, suspensions, syrup, suppositories, tablet, troches, lozenges, solution, buccal patch, thin film, oral gel, chewable tablet, chewing gum, and single-use syringes. Such individually packaged units may be combined in a package made of one or more of paper, cardboard, paperboard, metal foil and plastic foil, for example a blister pack. One or more administration units may be administered once or several times a day. One or more administration units may be administered three times a day. One or more administration units may be administered twice a day. One or more administration units may be administered on a first day and one or more administration units may be administered on the following days.

Avacopan has the formula:

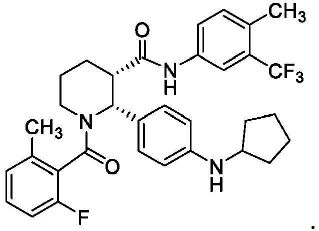

### Examples

The following examples are offered for illustrative purposes, and are not intended to limit the invention in any manner. Those of skill in the art will readily recognize a variety of noncritical parameters which can be changed or modified to yield essentially the same results.

### Example 1: Rapid Reduction of Urinary sCD163 correlates with Clinical Benefit of C5aR Inhibitor Avacopan in ANCA-Associated Vasculitis

The clinical trial results presented herein comprised 3 patient groups: (1) Full dose prednisone (60 mg), standard of care; (2) Avacopan 30 mg b.i.d. plus low dose prednisone (20 mg); (3) Avacopan 30 mg b.i.d. plus no prednisone. All patients received either IV cyclophosphamide or IV rituximab. sCD163 (ELISA) and creatinine were measured pre-dose, and at days 8, 15, 29, 57, and 85. The groupings are further described below in Table 1.

**Table 1: Treatment Groups**

| **Group A (SOC) n=23** | **Group B n=22** | **Group C n=22** |
|---|---|---|
| Placebo of Avacopan for 84 days | Avacopan 30 mg BID for 84 days | Avacopan 30 mg BID for 84 days |
| Prednisone 60 mg QD tapered over 140 days | Prednisone 20 mg QD tapered over 98 days | Placebo of Prednisone |
| Cyclophosphamide 15 mg/kg IV on Days 1, 15, 29, 57 and 85 **or** Rituximab 375 mg/m² IV on Days 1, 8, 15, and 22 | | |
| All subjects were followed up for additional 12 weeks after completion of Avacopan/placebo | | |

The goal of this study was to evaluate the effectiveness of avacopan in humans with AAV and the evaluate the effect of three different therapeutic regimens on urinary sCD163 and to investigate the correlation between urinary sCD163 levels and the following renal function parameters: eGFR, UACR, and inflammation marker urinary MCP-1/creatinine ratio. CD163 is a glycosylated membrane protein exclusively expressed on monocytes and macrophages. Membrane bound CD163 can be enzymatically cleaved to form soluble CD163 (sCD163) via ectodomain shredding in response to proinflammatory stimuli. Urinary sCD163 levels were reported to be elevated in active renal vasculitis compared to the levels in patentis in remission and healthy controls. Increased urinary levels of sCD163 in active AAV is associated with higher numbers of CD163 positive cells in the kidney.

### Materials and Methods

Serum samples were collected using serum separation blood tubes (SST) and shipped to the central laboratory refrigerated the same day and analyzed for creatinine and eGFR (calculated using MDRD serum creatinine equation).

Mid-stream urine samples were collected with a clean catch method and were shipped to the central laboratory refrigerated the same day for analysis of creatinine, albumin and UACR.

Another fraction of urine was centrifuged at 1200 g and 2-8°C for 10 minutes. Supernatant was aliquoted into cryovials and stored at -70°C until analysis.

Urine sCD163 and MCP-1 were analyzed by ELISA in complete subject sets using ELISA kits from R&D systems.

Urine sCD163/creatinine ratios, UACR, and urine MCP-1/creatinine ratios were log transformed for normal distribution.

Change from baseline in urine sCD163/creatinine ratios for each time point within group was analyzed by the mixed effects model for repeated measures. P<0.05 was considered as statistically significant.

The correlation of urine sCD163/creatinine ratio to eGFR, UACR, and urine MCP-1/creatinine ratio was analyzed by the regression model for repeated measures

As shown in Table 2, Avacopan treatment reduced sCD163/creatinine markedly by day 8, and further over the course of 12-weeks.

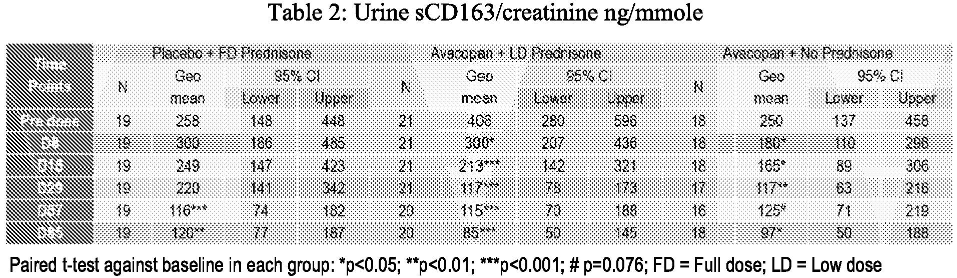

By contrast, standard of care controls with full-dose prednisone therapy did not show improvement in urinary sCD163 until day 57. The urinary sCD163/creatinine levels were highly correlative with previously reported improvements in urinary albumin/creatinine ratio and MCP-1/creatinine ratio (p<0.0001).

**FIG. 1A-E** illustrates the rapid decrease in diseases activity and significant improvement in health-related quality of life in patients treated with Avacopan. Panel **A** plots the Birmingham Vasculitis Activity Score; Panel **B** plots the Urinary Albumin: creatinine Ratio; Panel **C** plots the Health-related quality-of-life measurement EQ-5D-5L visual analog scale , expressed as mean +/- SEM over the treatment period; Panel **D** and Panel **E** plot the health-related quality-of-life measurement Medical Outcomes Study SF-36 version 2 Physical Functioning and Role Emotional components, expressed as mean +/- SEM over the treatment period.

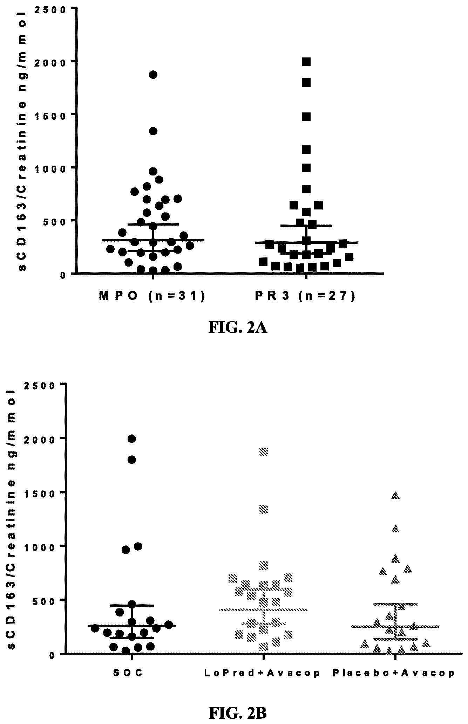

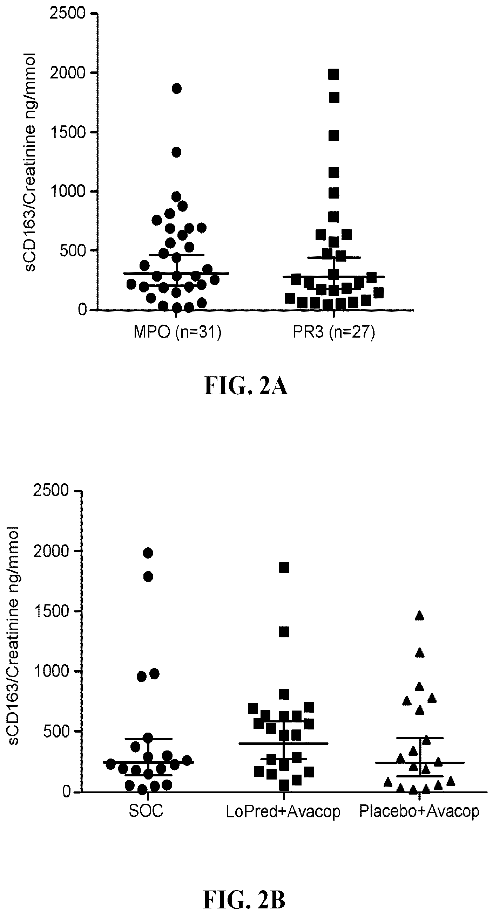

**FIG. 2A-B** illustrates urinary sCD163/creatinine ratios at baseline. **FIG. 2A** shows levels of urinary sCD163/creatinine ratios in patients with anti-MPO and anti-PR3 autoantibodies; **FIG. 2B** shows the levels of urnary sCD163/creatinine ratios in patients randomized to three different treatment groups. Urinary sCD163/creatinine ratios at baseline were comparable in patients with anti-MPO and anti-PR3 auto-antibodies and among three treatment groups.

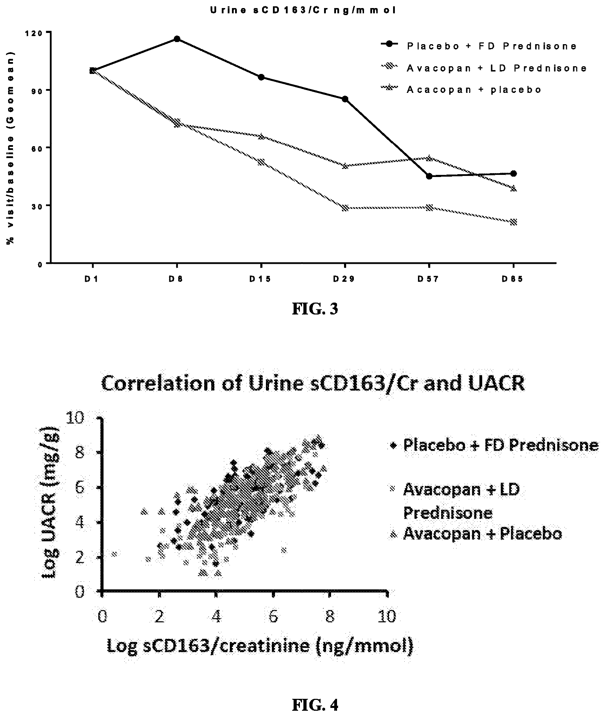

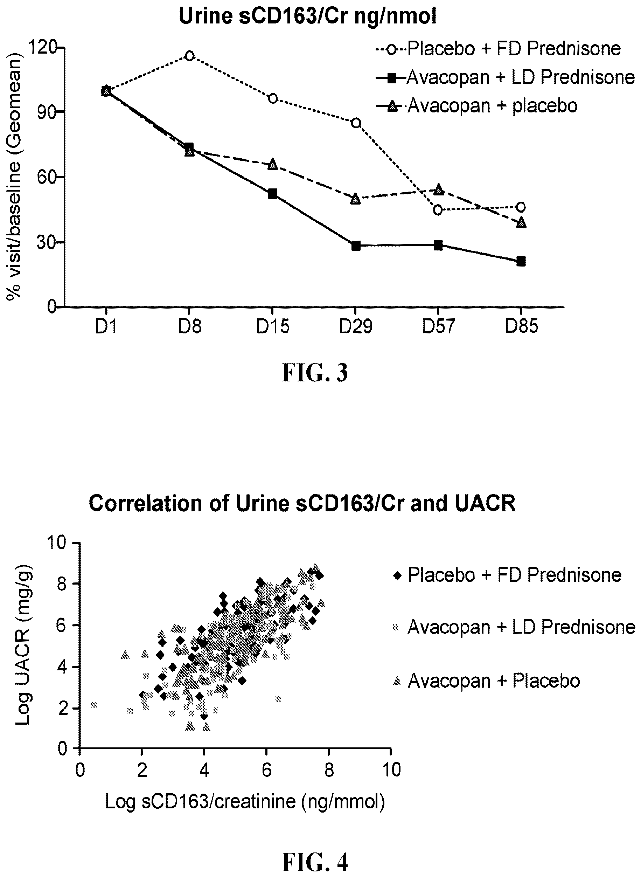

**FIG.** 3 illustrates the rapid reduction of urinary sCD163/creatinine ratio in patients treated with Avacopan. As shown in the graph, *p<0.05; **p<0.01; ***p<0.001; # p=0.076 is compared to baseline in each group.

**FIG. 4** illustrates the strong positive correction between urine sCD163/Cr and UACR. P<0.0001 for correlation; data from all subject and time points are included in the analysis.

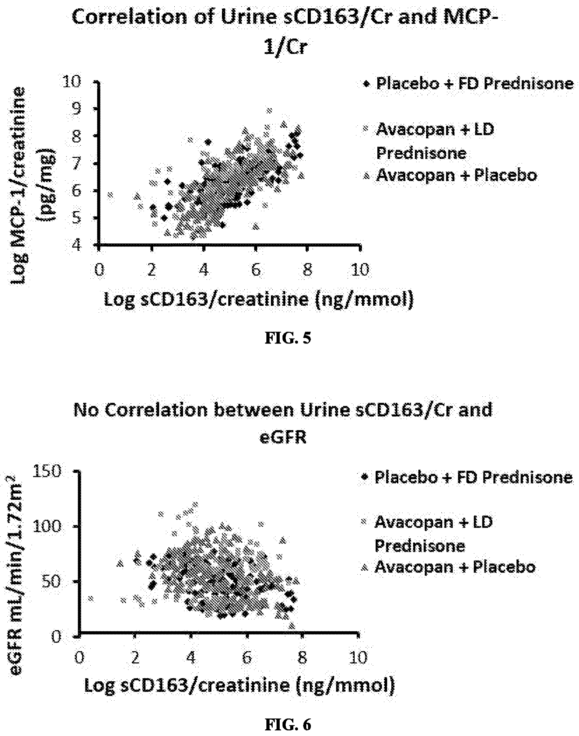

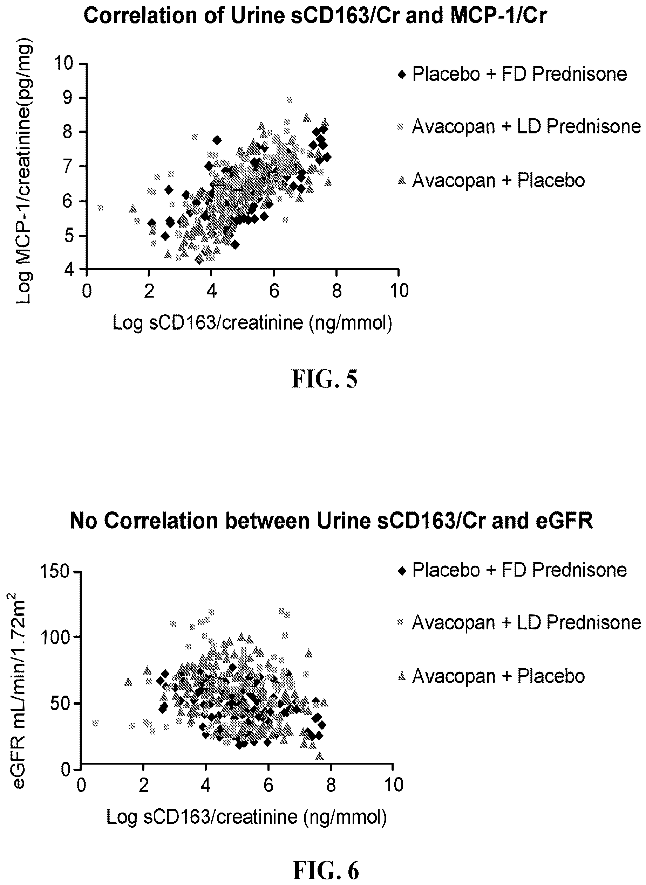

**FIG. 5** illustrates the strong positive correction between urine sCD163/Cr and MCP-1/Cr. P<0.0001 for correlation; data from all subject and time points are included in the analysis.

**FIG. 6** illustrates the lack of correction between urine sCD163/Cr and eGFR. P=0.655 for correlation; data from all subject and time points are included in the analysis.

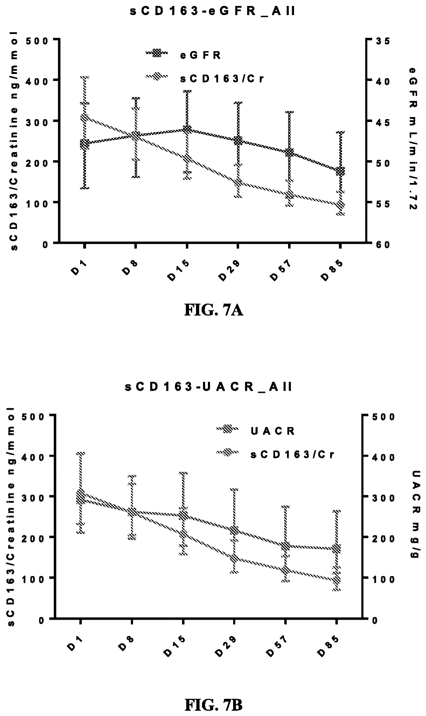

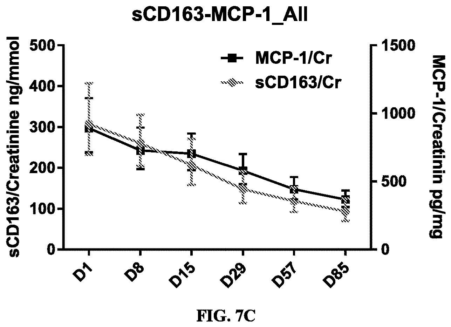

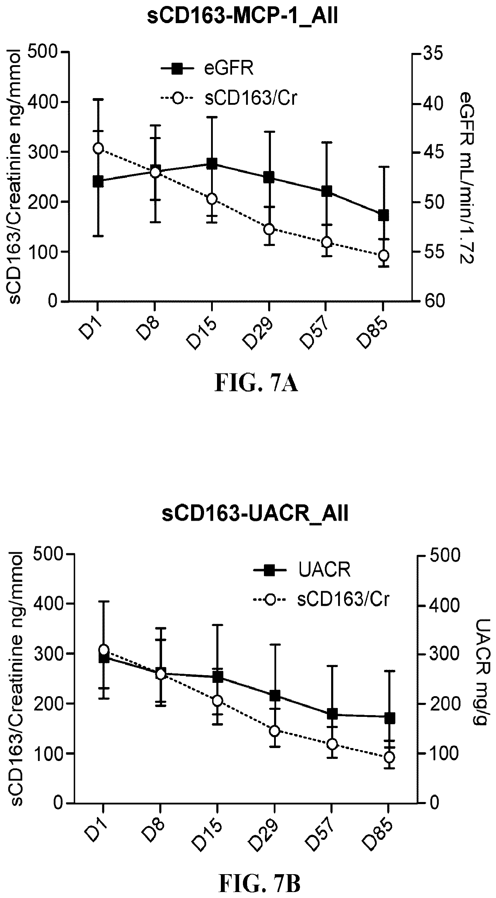

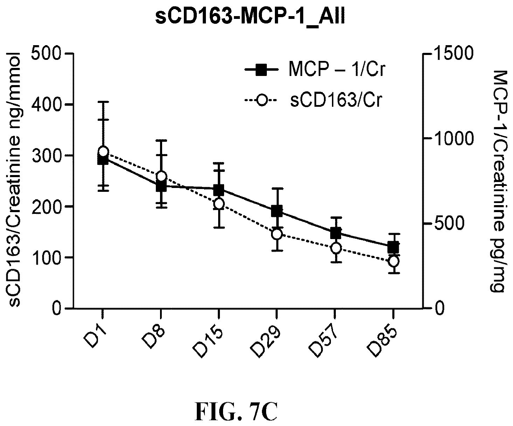

**FIG. 7A-C**illustrates the changes from baseline urine sCD163/Cr, eGFR, UACR, and MCP-1/Cr over time in all subjects. **FIG.** 7A plots eGFR and sCD163/Cr over time; **FIG. 7B** plots sCD163/Cr and UACR over time; **FIG.** 7C plots sCD163/Cr and MCP-1/Cr over time. As seen in the figures, the decrease in sCD163/Cr occurred before eGFR improvement, the decrease in sCD163/Cr occurred prior to decrease in UACR, and the decreases in sCD163/Cr and MCP-1/Cr correlated well temporally.

Avacopan administration led to a rapid reduction of sCD163, which correlated with rapid improvements of kidney inflammation markers, and AAV signs and symptoms in the trial. The significant positive correlation and temporal correlation of urine sCD163/Cr and MCP-1/Cr support urine sCD 163 as a marker of renal inflammation in patients with AAV.
The following numbered aspects form part of the present disclosure
**1.** A method for treating ANCA-associated vasculitis (AAV) with renal involvement in an individual in need thereof comprising administering a complement component 5a receptor (C5aR) antagonist to the individual if the individual exhibits an elevated urinary soluble CD163 (sCD163) to creatinine ratio compared to individuals without AAV.
**2.** A method for assessing the effectiveness of treatment with a complement component 5a receptor (C5aR) antagonist in an individual having ANCA-associated vasculitis (AAV), the method comprising:
   measuring the soluble CD163 (sCD163) to creatinine ratio in a urinary sample from the individual prior and subsequent to administration of the C5aR antagonist, wherein treatment is effective if the concentration of urinary sCD163 decreases subsequent to administration.
**3.** A method for reducing the concentration of urinary soluble CD163 (sCD163) in an individual diagnosed with ANCA-associated vasculitis (AAV), the method comprising:
   administering a complement component 5a receptor (C5aR) antagonist to the individual, wherein administration of the C5aR antagonist decreases the concentration of urinary sCD163 in a urinary sample provided by the individual.
**4.** The method of any one of **1-2,** wherein the urinary sCD163 to creatinine ratio is measured relative to the ratio of albumin to creatinine in a urinary sample from the individual.
**5.** The method of any one of **1-2,** wherein the urinary sCD163 to creatinine ratio is measured relative to the ratio of monocyte chemoattractant protein-1 (MCP-1) to creatinine in a urinary sample from the individual.
**6.** The method of **1 or 3,** wherein the concentration of sCD163 in a urinary sample from the individual after 8 days of administering a C5aR antagonist is reduced by at least 25 %.
**7.** The method of **2,** wherein an effective treatment decreases the sCD163 to creatinine ratio in a urinary sample from the individual subsequent to administration of the C5aR antagonist by at least 50% as compared to the sCD163 to creatinine ratio in a urinary sample from the individual prior to administration of the C5aR antagonist.
**8.** The method of any one of **1-5,** wherein the C5aR antagonist is Avacopan.
**9.** The method of any one of **1-5,** wherein the C5aR antagonist is 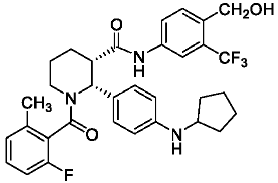 or a pharmaceutically acceptable salt thereof.
**10.** The method of any one of **1** to **9** wherein the C5aR antagonist is administered twice daily.
**11.** The method of any one of **1** to **9** wherein the C5aR antagonist is administered once a day.
**12.** The method of any one of **1** to **11** wherein the C5aR antagonist is administered orally.
**13.** The method of any one of **1** to **11** wherein 30 mg of the C5aR antagonist is administered twice daily.
**14.** The method of any one of **1** to **11** wherein 30 mg of the C5aR antagonist is administered twice daily via oral administration.
**15.** The method of any one of **1** to **14** wherein the individual is a human.
**16.** The method of any one of **1** to **15** wherein the individual receives treatment for 12 weeks.
**17.** The method of any one of **1** to **15** wherein the individual receives treatment for 26 weeks.
**18.** The method of any one of **1** to **15** wherein the individual receives treatment for 52 weeks.
**19.** The method of any one of **1** to **15** wherein the individual receives chronic treatment.
**20.** A method for treating ANCA-associated vasculitis (AAV) in an individual in need thereof comprising administering an effective amount of complement component 5a receptor (C5aR) antagonist, thereby treating ANCA-associated vasculitis.
**21.** The method of **20,** wherein the individual has AAV with renal involvement.
**22.** The method of **20** or **21,** wherein the C5aR antagonist is Avacopan.
**23.** The method of **20** or **21,** wherein the C5aR antagonist is 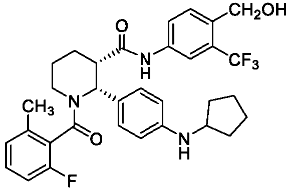 or a pharmaceutically acceptable salt thereof.
**24.** The method of any one of **20** to **23** wherein the C5aR antagonist is administered twice daily.
**25.** The method of any one of **20** to **23** wherein the C5aR antagonist is administered once a day.
**26.** The method of any one of **20** to **23** wherein the C5aR antagonist is administered orally.
**27.** The method of any one of **20** to **23** wherein 30 mg of the C5aR antagonist is administered twice daily.
**28.** The method of any one of **20** to **23** wherein 30 mg of the C5aR antagonist is administered twice daily via oral administration.
**29.**The method of any one of **20** to **28,** wherein the urinary albumin to creatinine ratio in the individual is reduced by at least 30% after 4 weeks of treatment as compared to the urinary albumin to creatinine ratio of the individual prior to treatment.
**30.**The method of any one of **20** to **28,** wherein the urinary albumin to creatinine ratio in the individual is reduced by at least 40% after 4 weeks of treatment as compared to the urinary albumin to creatinine ratio of the individual prior to treatment.
**31.** The method of any one of **20** to **28,** wherein the urinary albumin to creatinine ratio in the individual is reduced by at least 45% after 4 weeks of treatment as compared to the urinary albumin to creatinine ratio of the individual prior to treatment.
**32.** The method of any one of **20** to **28,** wherein the Birmingham vasculitis activity score of the individual is reduced by at least 50% after 4 weeks of treatment as compared to the Birmingham vasculitis activity score of the individual prior to treatment.
**33.** The method of any one of **20** to **28,** wherein the Birmingham vasculitis activity score of the individual is reduced by at least 60% after 4 weeks of treatment as compared to the Birmingham vasculitis activity score of the individual prior to treatment.
**34.** The method of any one of **20** to **33** wherein the individual receives treatment for 12 weeks.
**35.** The method of any one of **20** to **33** wherein the individual receives treatment for 26 weeks.
**36.** The method of any one of **20** to **33** wherein the individual receives treatment for 52 weeks.
**37.** The method of any one of **20** to **33** wherein the individual receives chronic treatment.

## Claims

1. A method for assessing the effectiveness of treatment with avacopan, having the formula: 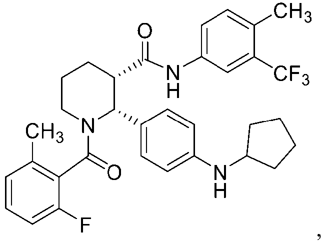 in an individual having ANCA-associated vasculitis (AAV), the method comprising:
measuring the soluble CD163 (sCD163) to creatinine ratio in a urinary sample from the individual prior and subsequent to administration of avacopan.

2. Avacopan, having the formula: 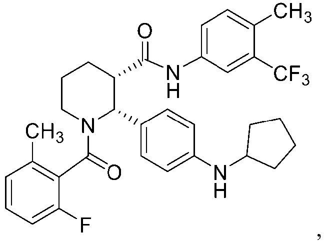 for use in a method of treating an individual having ANCA-associated vasculitis (AAV), the method comprising:
assessing the effectiveness of treatment by measuring the soluble CD163 (sCD163) to creatinine ratio in a urinary sample from the individual prior and subsequent to administration of avacopan.

3. The method of claim 1, or the avacopan for use of claim 2, wherein the urinary sCD163 to creatinine ratio is measured relative to the ratio of albumin to creatinine in a urinary sample from the individual.

4. The method of claim 1, or the avacopan for use of claim 2, wherein the urinary sCD163 to creatinine ratio is measured relative to the ratio of monocyte chemoattractant protein-1 (MCP-1) to creatinine in a urinary sample from the individual.

5. The method, or the avacopan for use, of any one of claims 1 to 4, wherein the avacopan is administered twice daily.

6. The method, or the avacopan for use, of any one of claims 1 to 4, wherein the avacopan is administered once a day.

7. The method, or the avacopan for use, of any one of claims 1 to 6, wherein the avacopan is administered orally.

8. The method, or the avacopan for use, of any one of claims 1 to 7, wherein the individual is a human, and the human receives treatment for 12 weeks.

9. The method, or the avacopan for use, of any one of claims 1 to 7, wherein the individual is a human, and the human receives treatment for 26 weeks.

10. The method, or the avacopan for use, of any one of claims 1 to 7, wherein the individual is a human, and the human receives treatment for 52 weeks.

11. The method, or the avacopan for use, of any one of claims 1 to 7, wherein the individual is a human, and the human receives chronic treatment.

12. The method, or the avacopan for use, of any one of claims 1 to 11, wherein treatment is effective if the concentration of urinary sCD163 decreases subsequent to administration.
